# EUROPEAN PATENT SPECIFICATION

(11) **EP 2 345 454 B1**
(45) Date of publication and mention of the grant of the patent: **15.05.2013**
(21) Application number: 11000203.7
(22) Date of filing: 12.01.2011
(51) Int. Cl.: A61N 5/04, A61B 18/18, A61B 18/00, A61B 19/00

(54) **Ablation device with user interface at device handle and system including same**
Ablationsvorrichtung mit Benutzerschnittstelle am Vorrichtungsgriff und System mit der Ablationsvorrichtung
Dispositif d'ablation avec une interface utilisateur sur la poignée et système l'incluant

(30) Priority: 13.01.2010 US 686726
(43) Date of publication of application: 20.07.2011
(73) Proprietor: Vivant Medical, Inc., Boulder CO 80301 (US)
(72) Inventor: Brannan, Joseph D., Erie, CO 80516 (US)
(74) Representative: Schmidt, Steffen

(56) References cited:
- WO-A1-2004/007023
- WO-A1-2004/042546
- WO-A2-02/085451
- US-A1- 2008 183 251

## Description

### BACKGROUND

### 1. Technical Field

The present disclosure relates to electrosurgical devices suitable for use in tissue ablation applications and, more particularly, to ablation devices with a user interface at the device handle and systems including the same.

### 2. Discussion of Related Art

Treatment of certain diseases requires the destruction of malignant tissue growths, e.g., tumors. Electromagnetic radiation can be used to heat and destroy tumor cells. Treatment may involve inserting ablation probes into tissues where cancerous tumors have been identified. Once the probes are positioned, electromagnetic energy is passed through the probes into surrounding tissue.

In the treatment of diseases such as cancer, certain types of tumor cells have been found to denature at elevated temperatures that are slightly lower than temperatures normally injurious to healthy cells. Known treatment methods, such as hyperthermia therapy, heat diseased cells to temperatures above 41° C while maintaining adjacent healthy cells below the temperature at which irreversible cell destruction occurs. These methods involve applying electromagnetic radiation to heat, ablate and/or coagulate tissue. Microwave energy is sometimes utilized to perform these methods. Other procedures utilizing electromagnetic radiation to heat tissue also include coagulation, cutting and/or ablation of tissue.

Electrosurgical devices utilizing electromagnetic radiation have been developed for a variety of uses and applications. A number of devices are available that can be used to provide high bursts of energy for short periods of time to achieve cutting and coagulative effects on various tissues. There are a number of different types of apparatus that can be used to perform ablation procedures. Typically, microwave apparatus for use in ablation procedures include a microwave generator that functions as an energy source, and a microwave surgical instrument (e.g., microwave ablation probe) having an antenna assembly for directing the energy to the target tissue. The microwave generator and surgical instrument are typically operatively coupled by a cable assembly having a plurality of conductors for transmitting microwave energy from the generator to the instrument, and for communicating control, feedback and identification signals between the instrument and the generator.

There are several types of microwave probes in use, e.g., monopole, dipole and helical, which may be used in tissue ablation applications. ln monopole and dipole antenna assemblies, microwave energy generally radiates perpendicularly away from the axis of the conductor. Monopole antenna assemblies typically include a single, elongated conductor. A typical dipole antenna assembly includes two elongated conductors that are linearly aligned and positioned end-to-end relative to one another with an electrical insulator placed therebetween. Helical antenna assemblies include helically-shaped conductor configurations of various dimensions, e.g., diameter and length. The main modes of operation of a helical antenna assembly are normal mode (broadside), in which the field radiated by the helix is maximum in a perpendicular plane to the helix axis, and axial mode (end fire), in which maximum radiation is along the helix axis.

A microwave transmission line typically includes a long, thin inner conductor that extends along the longitudinal axis of the transmission line and is surrounded by a dielectric material and is further surrounded by an outer conductor around the dielectric material such that the outer conductor also extends along the transmission line axis. ln one variation of an antenna, a waveguiding structure, such as a length of transmission line or coaxial cable, is provided with a plurality of openings through which energy "leaks" or radiates away from the guiding structure. This type of construction is typically referred to as a "leaky coaxial" or "leaky wave" antenna.

Cooling the ablation probe may enhance the overall heating pattern of the antenna, prevent damage to the antenna and prevent harm to the clinician or patient. Because of the small temperature difference between the temperature required for denaturing malignant cells and the temperature normally injurious to healthy cells, a known heating pattern and precise temperature control is needed to lead to more predictable temperature distribution to eradicate the tumor cells while minimizing the damage to surrounding normal tissue.

During certain procedures, it can be difficult to assess the extent to which the microwave energy will radiate into the surrounding tissue, making it difficult to determine the area or volume of surrounding tissue that will be ablated. Ablation volume is correlated to antenna design, antenna performance, antenna impedance, ablation time and wattage, and tissue characteristics, e.g., tissue impedance. The particular type of tissue ablation procedure may dictate a particular ablation volume in order to achieve a desired surgical outcome. By way of example and without limitation, a spinal ablation procedure may call for a longer, narrower ablation volume, whereas in a prostate ablation procedure a more spherical ablation volume may be required.

In some instances, targeted lesions may be located on or near the surface of the target organ. Some ablation targeted lesions are too small or too hard to be punctured by an ablation probe. ln these cases, doctors may place the probe as close as possible to the lesion and perform an ablation. With non-directional ablation probes, the ablation may radiate to both sides of the probe.

Treatment of certain tumors may involve probe repositioning during the ablation procedure, such as where the tumor is larger than the probe or has a shape that does not correspond with available probe geometry or radiation pattern. The surgeon, before or after treatment is completed, may remove the probe from tissue while power is delivered to the probe antenna and an unintentional radiation exposure may occur.

US 2008/183251 A1 discloses an apparatus for non-invasive treatment by application of RF energy. In one embodiment, the apparatus comprises a controller inputted by a temperature signal measured by a temperature sensor so as to interrupt the application of RF energy when a temperature threshold is exceeded. Other embodiments disclose IR detectors (i.e. radiation detectors).

WO 02/085451 A2 discloses an electric device for treating the skin of a subject. In some embodiments, WO 02/085451 A2 discloses apparatuses for transmitting drugs to the skin of a patient. In some other embodiments, WO 02/085451 A2 discloses, in general terms, a pressure sensor for controlling the drug supply. In another embodiment, WO 02/085451 A2 discloses a drug reservoir 342.

WO 2004/042546 A1 discloses a handheld imaging system.

WO 2004/007023 A1 discloses a handpiece for tissue treatment using a light source. The handpiece only has an optical interconnection to the light source and no electrical interconnection to the light source. The handpiece comprises means for receiving a first light beam emitted from a light source, a sensor for measuring beam parameters of the first light beam, processing means for analyzing the measured beam parameters, and for producing one or more corresponding output, and means for deflecting the first light beam into a treating light beam and for directing the treating light beam towards a target area so that the handpiece is adapted to be operated on the basis of the one or more produced output from the processing means. Hereby, electrical adaptation between handpiece and light source is avoided.

### SUMMARY

The invention is defined in claim 1. The present disclosure relates to an ablation device including a handle assembly including a distal end, an ablation probe extending distally from the distal end of the handle assembly, and a user interface disposed at the handle assembly. The ablation probe is operably coupled to the user interface. The user interface includes a controller unit, a power on/off switch, and at least one sensor capable of generating an electrical signal. The controller is in communication with the at least one detector and configured to override operation of the power on/off switch in response to the electrical signal generated by the at least one detector.

The present disclosure also relates to a system for ablating tissue including an energy source and an ablation device. The ablation device includes a handle assembly, a user interface disposed on the handle assembly, and a probe operably coupled to the user interface extending distally from a distal end of the handle assembly.

### BRIEF DESCRIPTION OF THE DRAWINGS

Objects and features of the presently disclosed ablation device with a user interface at the handle and system including the same will become apparent to those of ordinary skill in the art when descriptions of various embodiments thereof are read with reference to the accompanying drawings, of which:

FIG. 1 is a schematic diagram of an ablation system in accordance with an embodiment of the present disclosure;

FIG. 2 is a partial, longitudinal cross-sectional view of an embodiment of the energy applicator of the ablation system shown in FIG. 1 in accordance with the present disclosure;

FIG. 3 is a bottom perspective view of the ablation device of FIG. 1 according to an embodiment of the present disclosure;

FIG.4 is a state diagram according to an embodiment of the present disclosure;

FIG. 5 is a perspective view of another embodiment of an ablation device with a user interface at the handle in accordance with the present disclosure;

FIG. 6 is a perspective view of yet another embodiment of an ablation device with a user interface at the handle in accordance with the present disclosure;

FIG. 7 is a schematic diagram of a feedback control system in accordance with an embodiment of the present disclosure;

FIG. 8 is a perspective view of an embodiment of an ablation device with a user interface at the handle in accordance with the present disclosure that includes a male connector disposed at the proximal end of the energy applicator;

FIG.9 is a perspective view of an embodiment of a directional reflector assembly in accordance with the present disclosure that includes a tubular portion having a female connector adapted for attachment to the male connector of the ablation device of FIG. 8;

FIG. 10 is a perspective view of the ablation device of FIG. 9 shown with the directional reflector assembly of FIG. 9 mounted on the probe thereof; and

FIG. 11 is a flowchart illustrating a method of ablating tissue.

### DETAILED DESCRIPTION

Hereinafter, embodiments of the presently disclosed ablation device with a handle user interface, system including the same, and method of ablating tissue using the same will be described with reference to the accompanying drawings. Like reference numerals may refer to similar or identical elements throughout the description of the figures. As shown in the drawings and as used in this description, and as is traditional when referring to relative positioning on an object, the term "proximal" refers to that portion of the apparatus that is closer to the user and the term "distal" refers to that portion of the apparatus that is farther from the user.

This description may use the phrases "in an embodiment," "in embodiments," "in some embodiments," or "in other embodiments," which may each refer to one or more of the same or different embodiments in accordance with the present disclosure. For the purposes of this description, a phrase in the form "A/B" means A or B. For the purposes of the description, a phrase in the form "A and/or B" means "(A), (B), or (A and B)". For the purposes of this description, a phrase in the form "at least one of A, B, or C" means "(A), (B), (C), (A and B), (A and C), (B and C), or (A, B and C)".

Electromagnetic energy is generally classified by increasing energy or decreasing wavelength into radio waves, microwaves, infrared, visible light, ultraviolet, X-rays and gamma-rays. As it is used in this description, "microwave" generally refers to electromagnetic waves in the frequency range of 300 megahertz (MHz) (3 x 10⁸ cycles/second) to 300 gigahertz (GHz) (3 x **10¹¹** cycles/second). As it is used in this description, "ablation procedure" generally refers to any ablation procedure, such as microwave ablation, radio frequency (RF) ablation or microwave ablation assisted resection. As it is used in this description, "energy applicator" generally refers to any device that can be used to transfer energy from a power generating source, such as a microwave or RF electrosurgical generator, to tissue.

As it is used in this description, "transmission line" generally refers to any transmission medium that can be used for the propagation of signals from one point to another. As it is used in this description, "switch" or "switches" generally refers to any electrical actuators, mechanical actuators, electro-mechanical actuators (rotatable actuators, pivotable actuators, toggle-like actuators, buttons, etc.) or optical actuators.

As it is used in this description, "length" may refer to electrical length or physical length. ln general, electrical length is an expression of the length of a transmission medium in terms of the wavelength of a signal propagating within the medium. Electrical length is normally expressed in terms of wavelength, radians or degrees. For example, electrical length may be expressed as a multiple or sub-multiple of the wavelength of an electromagnetic wave or electrical signal propagating within a transmission medium. The wavelength may be expressed in radians or in artificial units of angular measure, such as degrees. The electric length of a transmission medium may be expressed as its physical length multiplied by the ratio of (a) the propagation time of an electrical or electromagnetic signal through the medium to (b) the propagation time of an electromagnetic wave in free space over a distance equal to the physical length of the medium. The electrical length is in general different from the physical length. By the addition of an appropriate reactive element (capacitive or inductive), the electrical length may be made significantly shorter or longer than the physical length.

Various embodiments of the present disclosure provide ablation devices with a user interface at the handle assembly (also referred to herein as a handle user interface) for treating tissue and methods of directing electromagnetic radiation to tissue. Embodiments may be implemented using electromagnetic radiation at microwave frequencies or at other frequencies. An electrosurgical system including an ablation device with a handle user interface, according to various embodiments, is designed and configured to operate between about 500 MHz and about 10 GHz, and may provide a directional radiation pattern.

Various embodiments of the presently disclosed ablation devices and electrosurgical system including the same are suitable for microwave ablation and for use to pre-coagulate tissue for microwave ablation assisted surgical resection. Although various methods described hereinbelow are targeted toward microwave ablation and the complete destruction of target tissue, it is to be understood that methods for directing electromagnetic radiation may be used with other therapies in which the target tissue is partially destroyed or damaged, such as, for example, to prevent the conduction of electrical impulses within heart tissue. ln addition, although the following description describes the use of a dipole microwave antenna, the teachings of the present disclosure may also apply to a monopole, helical, or other suitable type of microwave antenna.

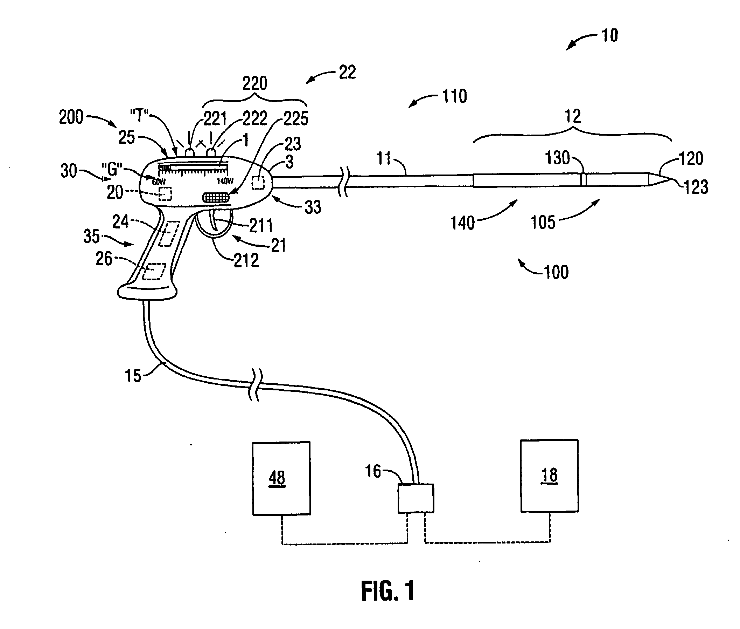

lt is envisioned that any combination of battery cells, a battery pack, fuel cell and/or high-energy capacitor may be used to provide power to the ablation device (e.g., 200, 500 and 600 shown in FIGS. 1, 5 and 6, respectively). For example, capacitors may be used in conjunction with a battery pack. ln such case, the capacitors may discharge a burst of power to provide energy more quickly than batteries are capable of providing, as batteries are typically slow-drain devices from which current cannot be quickly drawn. It is envisioned that batteries may be connected to the capacitors to charge the capacitors.

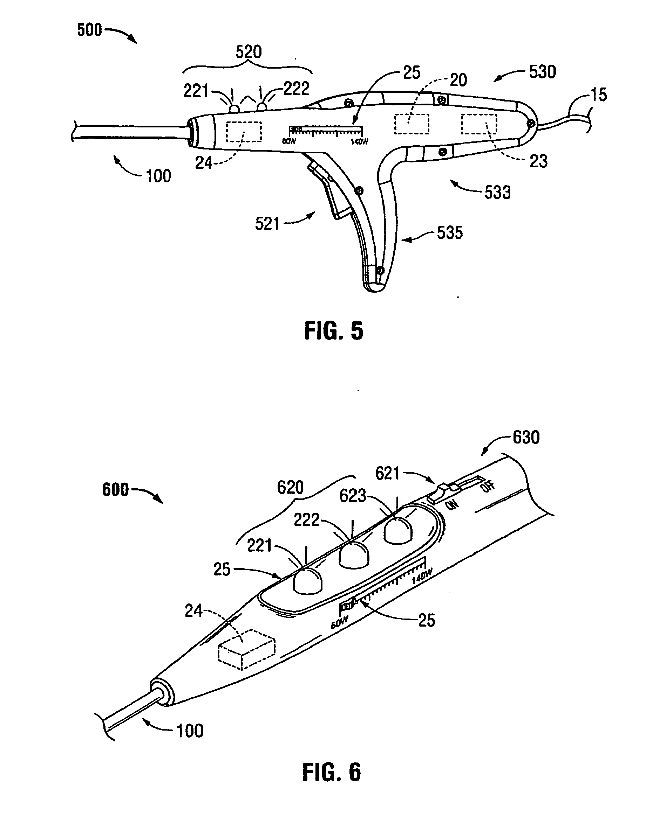

A battery pack may include at least one disposable battery. ln such case, the disposable battery may be between about 9 volts and about 30 volts, and may be useful as a backup power source for the controller 20. ln some embodiments, a transmission line (e.g., 15 shown in FIGS. 1 and 5) is provided to connect the ablation device to an electrosurgical power generating source (e.g., 48 shown in FIG. 1).

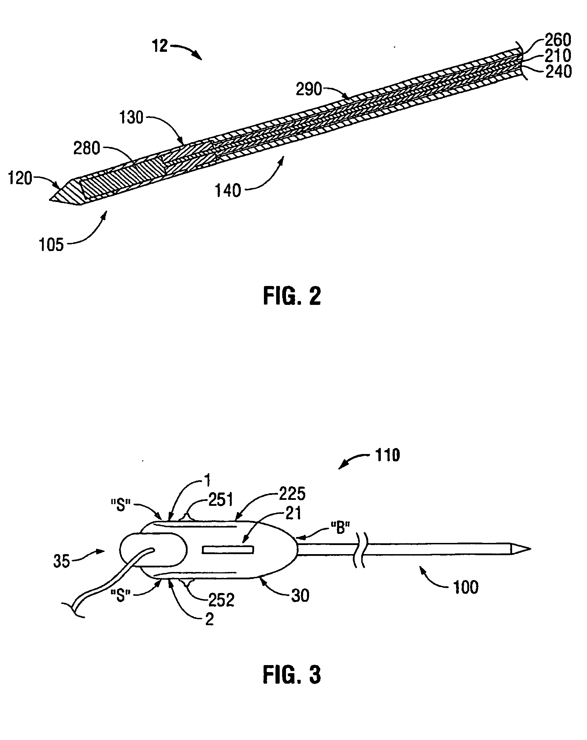

FIG. 1 shows an electrosurgical system 10, according to an embodiment of the present disclosure that includes an ablation device 110. Ablation device 110 generally includes a handle assembly 30 including a grip portion 35 and a handle body 33 configured to support an energy applicator or probe 100 at a distal end 3 thereof. As cooperatively shown in FIGS. 1 and 3, the handle body 33 includes a top portion "T", side portions "S" and a bottom portion "B". Probe 100, which is described in more detail later in this disclosure, generally includes an antenna assembly 12 having a radiating portion connected by a feedline 11 (or shaft) to the handle assembly 30. Antenna assembly 12 and the feedline 11 may have various dimensions, e.g., diameter and length. Handle assembly 30 according to various embodiments is adapted to electrically couple the feedline 11 to a transmission line 15. As shown in FIG. 1, the transmission line 15 may be coupled to a connector 16, which may further operably connect the probe 100 to an electrosurgical power generating source 48, e.g., a microwave or RF electrosurgical generator. ln some embodiments, a distal portion of the transmission line 15 may be disposed within the handle assembly 30, e.g., within the grip portion 35 and/or the handle body 33.

Ablation device 110 according to embodiments of the present disclosure includes a user interface (shown generally as 200 in FIG. 1) at the handle assembly 30. User interface 200 according to embodiments of the present disclosure includes a controller 20 and includes a power on/off trigger or switch 21, a radiation detector 24, and an indicator unit 22 adapted to provide a perceptible sensory alert, which may be an audio, visual, or other sensory alarm. Controller 20 may include any type of computing device, computational circuit, or any type of processor or processing circuit capable of executing a series of instructions that are stored in a memory (not shown) of the controller 20. Power on/off trigger or switch 21, the radiation detector 24 and/or the indicator unit 22 may be electrically coupled to the controller 20.

User interface 200 may additionally, or alternatively, include an intensity controller 25 adapted to allow the user to adjust the power parameters (e.g., voltage, power and/or current intensity) delivered to the probe 100. ln some embodiments, the user interface 200 may include a fluid-flow monitoring system 26 adapted to monitor and/or regulate the pressure and/or flow rate of fluid and capable of generating a signal indicative of an abnormal fluid circulation condition. ln some embodiments, the user interface 200 may include a reflected-power monitoring system 23 adapted to monitor power signals reflected from the probe 100. lntensity controller 25, the fluid-flow monitoring system 26 and/or the reflected-power monitoring system 23 may be electrically coupled to the controller 20.

Indicator unit 22 may include audio and/or visual indicator devices. lndicator unit 22 according to various embodiments includes an alarm or output component 220 that includes logic or circuitry to generate a signal when power is provided to the indicator unit 22. ln some embodiments, the indicator unit 22 is adapted to generate an audio signal and the output component 220 includes an audio circuit with a speaker 225. ln some embodiments, the indicator unit 22 is adapted to generate a visual signal and the output component 220 includes a light source, such as a light-emitting diode (LED). lndicator unit 22 may also include a display device (not shown), such as a flat panel display, e.g., a liquid crystal display (LCD), or other suitable display device, to provide information/feedback to a user.

In the embodiment illustrated in FIG. 1, the indicator unit 22 includes a first LED 221 and a second LED 222 in row configuration disposed on the top portion "T" of the handle body 33. It is envisioned that the first and second LEDs 221 and 222, respectively, provide information/feedback (e.g., visual feedback) to a user. For example, illumination of the first LED 221 may indicate that power is turned on, and illumination of the second LED 222 may indicate that power is turned off. First LED 221 and the second LED 222 may be uni- or multi-colored. ln an embodiment, the first LED 221 is a green LED, and the second LED 222 is a red LED. As can be appreciated, the first LED 221 may be a red LED, and the second LED 222 may be a green LED. The shape and size of the first and second LEDs 221 and 222, respectively, may be varied from the configuration depicted in FIG. 1. It is contemplated that a single LED may be utilized in place of the first and second LEDs 221 and 222.

Indicator unit 22 may additionally, or alternatively, include one or more audio indicator devices. As cooperatively shown in FIGS. 1 and 3, a speaker 225 may be disposed on a side portion "S" of the handle body 33. It is contemplated that any number and size of speakers may be incorporated into the handle body 33 and/or the grip portion 35. The positions of the first and second LEDs 221 and 222, respectively, and the speaker 225, e.g., in relation to the distal end 3 of the handle body 33, may be varied from the configuration depicted in FIG. 1.

User interface 200 according to various embodiments includes a power on/off switch 21. ln the embodiment illustrated in FIG. 1, the power on/off switch 21 includes a trigger 211 located within a trigger guard 212. The shape and size of the trigger 211 and the trigger guard 212 may be varied from the configuration depicted in FIG. 1. Power on/off switch 21 may utilize any suitable switch configuration. Examples of switch configurations that may be suitable for use with the ablation device 110 include, but are not limited to, pushbutton, toggle, rocker (e.g., 521 shown in FIG. 5), tactile, snap, rotary, slide (e.g., 621 shown in FIG. 6) and thumbwheel.

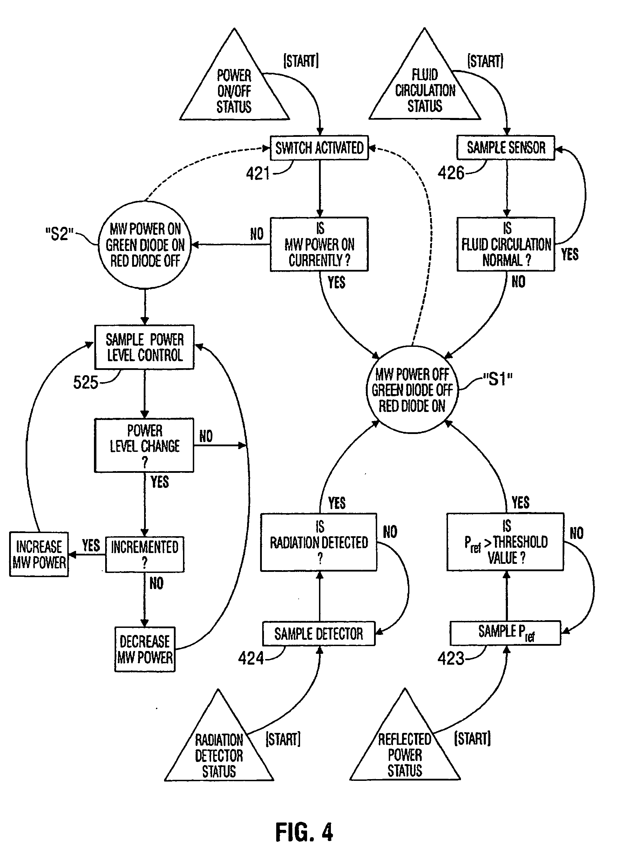

Radiation detector 24 according to various embodiments is electrically coupled to the controller 20, and may include any suitable device capable of detecting electromagnetic radiation and converting it to another form of energy such as electrical signals. ln some embodiments, the radiation detector 24 may include a radiation-absorbing layer and/or scintillator crystals. Examples of radiation detector embodiments are disclosed in commonly assigned U.S. Patent Application Serial No. 12/542,785 filed on August 18, 2009, entitled "MICROWAVE ABLATION ANTENNA RADIATION DETECTOR". ln the case where radiation is detected by the radiation detector 24, embodiments of the presently disclosed ablation devices transition to a power-off state (e.g., "S1" shown in FIG. 4). After taking corrective action when necessary, the user may activate the power on/off switch (e.g., 21 shown in FIG. 1) on the handle user interface to start, resume, or restart a procedure.

In some embodiments, the user interface 200 includes an intensity controller 25 slidingly supported on the handle body 33. As cooperatively shown in FIGS. 1 and 3, the intensity controller 25 includes a pair of nubs (e.g., 251, 252 shown in FIG. 3) that are slidingly supported, one each, in respective guide channels 1, 2 formed in the outer surface of the handle body 33. By providing nubs 251, 252 on either side "S" of the handle assembly 30, the intensity controller 25 may be easily manipulated by either hand of the user, allowing operation by a right- or a left-handed user.

Intensity controller 25 according to embodiments of the present disclosure is a slide-type potentiometer, wherein the nubs 251, 252 have a first position (e.g., a proximal-most position closest to the proximal end of the handle body 33) corresponding to a relative low-intensity setting, a second position (e.g., a distal-most position closest to the distal end 3 of the handle body 33) corresponding to a relative high-intensity setting, and a plurality of intermediate positions corresponding to intermediate intensity settings. As can be appreciated, the intensity settings from the proximal end to the distal end may be reversed as well, e.g., high to low. Nubs 251, 252 of the intensity controller 25 and the corresponding guide channels 1, 2 may be provided with a series of cooperating discrete positions or detents defining a series of positions for ease of selection of the output intensity from the low-intensity setting to the high-intensity setting. The series of cooperating discrete positions or detents may also provide the surgeon with a degree of tactile feedback.

Intensity controller 25 is configured to adjust the power parameters (e.g., voltage, power and/or current intensity) and/or the power verses impedance curve shape to affect the perceived output intensity. For example, the greater the lateral displacement of the nubs 251, 252 in a distal direction, the greater the level of the power parameters transmitted to the ablation device 110. Intensity settings may be preset and selected from a look-up table, e.g., based on a choice of electrosurgical instruments/attachments, desired surgical effect, surgical specialty and/or surgeon preference. The selection may be made automatically or selected manually by the user. The intensity values may be predetermined or adjusted by the user.

As shown in FIG. 1, the intensity controller 25 may include indicia such as, for example, graduation marks "G" and/or wattage levels (e.g., "80W" or "140W") provided thereon. The indicia may be etched, stamped, formed or the like, e.g., alongside the sides of the guide channels 1, 2 along which the nubs 251, 252 are slideably moveable. The design and location of the indicia may be varied from the configuration depicted in FIG. 1.

Intensity controller 25 may be adapted to provide a degree of tactile feedback, e.g., a tactile "click". Additionally, or alternatively, audible feedback may be produced from the intensity controller 25 (e.g., an audible click), from electrosurgical power generating source 48 (e.g., an audible tone) and/or from the speaker 225 (e.g., an audible tone). Examples of audible tones include beep tones, click or tick tones, hum tones, steady tones, pulsing tones, and voice.

A variety of intensity controller designs and different locations of the intensity controller on the handle assembly 30 may suitably be used. Examples of intensity controller embodiments are disclosed in commonly assigned U.S. Patent No. 7,156,844, entitled "ELECTROSURGICAL PENCIL WITH IMPROVED CONTROLS".

As an alternative to, or in addition to, the switch 21 and/or the intensity controller 25, the user interface 200 may include voice input technology, which may include hardware and/or software incorporated in the controller 20, or a separate digital module connected to the controller 20. The voice input technology may include voice recognition, voice activation, voice rectification, and/or embedded speech. The user may be able to control the operation of the ablation device in whole or in part through voice commands, e.g., freeing one or both of the user's hands for operating other instruments. Voice or other audible output may also be used to provide the user with feedback.

Fluid-flow monitoring system 26, according to embodiments of the present disclosure, includes a sensor that is in fluid communication with the probe 100 that senses the pressure and/or flow rate of fluid flow in and/or out of the probe 100. In some embodiments, the sensor is a mechanical sensor, such as a diaphragm or a piston. In other embodiments, the sensor is an electrical sensor, such as a temperature-measuring resistor or electrical transducer. Fluid-flow monitoring system 26 may include a flow sensor that is adapted to provide a measurement of the rate of fluid flow in and/or out of the probe 100 and/or conduit fluidly coupled the probe 100. Fluid-flow monitoring system 26 may include a pressure sensor that provides a measurement of the fluid pressure in the probe 100 and/or conduit fluidly coupled the probe 100. A change in color of an LED (e.g., 623 shown in FIG. 6) may be used to indicate an abnormal fluid circulation condition, or the rate at which an LED flashes may be used to indicate fluid-flow speed and/or pressure. Examples of fluid-flow monitoring system embodiments are disclosed in commonly assigned U.S. Patent Application Serial No. 12/568,972 filed on September 29, 2009, entitled "FLOW RATE MONITOR FOR FLUID COOLED MICROWAVE ABLATION PROBE", U.S. Patent Application Serial No. 12/566,299 filed on September 24, 2009, entitled "OPTICAL DETECTION OF INTERRUPTED FLUID FLOW TO ABLATION PROBE", and U.S. Patent Application Serial No. 12/569,685 filed on September 29, 2009, entitled "FLOW RATE MONITOR FOR FLUID COOLED MICROWAVE ABLATION PROBE".

Reflected-power monitoring system 23 is electrically coupled to the controller 20, and may include any suitable device capable of detecting power signals reflected back from probe 100. For example, energy may be reflected from ablated tissue and received by the antenna assembly 12. Energy not transferred to the antenna assembly 12 (e.g., when the antenna and feedline do not have matching impedances) may be reflected back towards the energy source. Reflected-power monitoring system 23 may include a power sensor to monitor forward and reflected power. The power sensor may measure the power output of the electrosurgical power generating source 48 that is utilized by the antenna assembly 12. Examples of power measurement system embodiments are disclosed in commonly assigned U.S. Patent Application Serial No. 12/242,102 filed on September 30, 2008, entitled "MICROWAVE ABLATION GENERATOR CONTROL SYSTEM". As described in more detail later in this disclosure, based on the comparison result of a comparison of a reflected power sample to a predetermined threshold value, the controller 20 of the handle user interface 200 may cause the ablation device 110 to transition to a power-off state.

User interface 200, according to embodiments of the present disclosure includes different colors and/or intensities of text on screen and/or on switches for further differentiation between the displayed items. User feedback may also be included in the form of pulsed patterns of light, acoustic feedback (e.g., buzzers, bells or beeps that may be sounded at selected time intervals), verbal feedback, and/or haptic vibratory feedback (such as an asynchronous motor or solenoids), for example. The visual, auditory or haptic feedback (e.g., level of vibratory feedback perceived by the user) may be increased or decreased in intensity. Additionally, switches may be positioned at different heights from one another and/or may included raised indicia or other textural features (e.g., concavity or convexity) to allow a user to depress or otherwise move an appropriate switch without the need to look at the user interface 200.

User interface 200 may include a separate display screen or screens and input devices (e.g., switches or buttons), or the input devices may be incorporated in whole or in part in screen. For example, a touch screen liquid crystal display (LCD) may be used to allow the user to provide input while viewing operational feedback. The touch screen LCD may include resistive, capacitive or surface acoustic wave controls. This approach may enable facilitation of sealing screen components to help sterilize the ablation device, as well as preventing particle and/or fluid contamination. In some embodiments, a display screen is pivotably or rotatably mounted to the ablation device for flexibility in viewing the screen during use or preparation. The display screen may be hinged or ball-and-socket mounted to the ablation device, for example.

In accordance with embodiments of the present disclosure, at least some of the information monitored by the various sensors, e.g., radiation detector 24 and fluid-flow monitoring system 26, in the ablation device 110 may be provided to a video screen or monitoring system in an operating room. For instance, the data may be transmitted to a receiver for the operating room monitoring system from a communication transmitter incorporated in or associated with the ablation device, via technology including Bluetooth^{®}, ANT3^{®}, KNX^{®}, Z-Wave^{®}, X10^{®}, Wireless USB, Wi-Fi^{®}, IrDA^{®}, NanoNet^{®}, TinyOS^{®}, ZigBee^{®}, 802.11 IEEE, and other radio, infrared, UHF, VHF communications. Such features may facilitate monitoring by the user of the ablation device 110 or other operating room or hospital personnel or remotely located persons.

Hereinbelow, embodiments of the presently disclosed probe 100 will be described with reference to FIGS. 1 and 2. Feedline 11 may be formed from any suitable flexible, semi-rigid or rigid microwave conductive cable and may connect directly to an electrosurgical power generating source 48. Alternatively, the feedline 11 may electrically connect the antenna assembly 12 via the transmission line 15 to the electrosurgical power generating source 48: Feedline 11 may have a variable length from a proximal end of the antenna assembly 12 to a distal end of transmission line 15 ranging from a length of about one inch to about twelve inches. Feedline 11 may be formed of suitable electrically conductive materials, e.g., copper, gold, silver or other conductive metals having similar conductivity values. Feedline 11 may be made of stainless steel, which generally offers the strength required to puncture tissue and/or skin. Conductive materials used to form the feedline 11 may be plated with other materials, e.g., other conductive materials, such as gold or silver, to improve their properties, e.g., to improve conductivity, decrease energy loss, etc. ln some embodiments, the feedline 11 includes stainless steel, and to improve the conductivity thereof, the stainless steel may be coated with a layer of a conductive material such as copper or gold. Feedline 11 may include an inner conductor, a dielectric material coaxially surrounding the inner conductor, and an outer conductor coaxially surrounding the dielectric material. Antenna assembly 12 may be formed from a portion of the inner conductor that extends distal of the feedline 11 into the antenna assembly 12. Feedline 11 may be cooled by fluid e.g., saline or water, to improve power handling, and may include a stainless steel catheter.

In some embodiments, the power generating source 48 is configured to provide microwave energy at an operational frequency from about 500 MHz to about 2500 MHz. ln other embodiments, the power generating source 48 is configured to provide microwave energy at an operational frequency from about 500 MHz to about 10 GHz. Power generating source 48 may be configured to provide various frequencies of electromagnetic energy. Transmission line 15 may additionally, or alternatively, provide a conduit (not shown) configured to provide coolant from a coolant source 18 to the probe 100.

Located at the distal end of the antenna assembly 12 is an end cap or tapered portion 120 that may terminate in a sharp tip 123 to allow for insertion into tissue with minimal resistance. The end cap or tapered portion 120 may include other shapes, such as, for example, a tip 123 that is rounded, flat, square, hexagonal, or cylindroconical.

**In** some variations, the antenna assembly 12 includes a distal radiating portion 105 and a proximal radiating portion 140. A junction member 130 may be provided. Junction member 130, or portions thereof, may be disposed between the proximal and distal radiating portions, 140 and 105, respectively. ln some embodiments, the distal and proximal radiating portions 105, 140 align at the junction member 130, which is generally made of a dielectric material, e.g., adhesives, and are also supported by the inner conductor that extends at least partially through the distal radiating portion 105. Junction member 130 may be formed from any suitable elastomeric or ceramic dielectric material by any suitable process. ln some embodiments, the junction member 130 is formed by over-molding and includes a thermoplastic elastomer, such as, for example, polyether block amide (e.g., PEBAX^{®}, manufactured by The Arkema Group of Colombes, France), polyetherimide (e.g., ULTEM^{®} and/or EXTEM^{®}, manufactured by SABIC Innovative Plastics of Saudi Arabia) and/or polyimide-based polymer (e.g., VESPEL^{®}, manufactured by E. I. du Pont de Nemours and Company of Wilmington, Delaware, United States). Junction member 130 may be formed using any suitable over-molding compound by any suitable process, and may include use of a ceramic substrate.

In some embodiments, the antenna assembly 12 may be provided with a coolant chamber (not shown). Additionally, the junction member 130 may include coolant inflow and outflow ports (not shown) to facilitate the flow of coolant into, and out of, the coolant chamber. Examples of coolant chamber and coolant inflow and outflow port embodiments are disclosed in commonly assigned U.S. Patent Application Serial No. 12/401,268 filed on March 10, 2009, entitled "COOLED DIELECTRICALLY BUFFERED MICROWAVE DIPOLE ANTENNA", and U.S. Patent No. 7,311,703, entitled "DEVICES AND METHODS FOR COOLING MICROWAVE ANTENNAS".

In some embodiments, the antenna assembly 12 may be provided with an outer jacket (not shown) disposed about the distal radiating portion 105, the junction 130 and/or the proximal radiating portion 140. The outer jacket may be formed of any suitable material, such as, for example, polymeric or ceramic materials. The outer jacket may be applied by any suitable method, such as, for example, heat shrinking, over-molding, coating, spraying dipping, powder coating, baking and/or film deposition. The outer jacket may be a water-cooled catheter formed of a material having low electrical conductivity.

During a procedure, e.g. an ablation procedure, using the electrosurgical system 10, the probe 100 is inserted into or placed adjacent to tissue and energy is supplied thereto. Ultrasound or computed tomography (CT) guidance may be used to accurately guide the probe 100 into the area of tissue to be treated. Probe 100 may be placed percutaneously or surgically, e.g., using conventional surgical techniques by surgical staff. A clinician may pre-determine the length of time that microwave energy is to be applied. Application duration may depend on many factors such as tumor size and location and whether the tumor was a secondary or primary cancer. The duration of microwave energy application using the probe 100 may depend on the progress of the heat distribution within the tissue area that is to be destroyed and/or the surrounding tissue. Single or multiple probes 100 may provide ablations in short procedure times, e.g., a few minutes, to destroy cancerous cells in the target tissue region.

A plurality of probes 100 may be placed in variously-arranged configurations to substantially simultaneously ablate a target tissue region, making faster procedures possible. Multiple probes 100 may be used to synergistically create a large ablation or to ablate separate sites simultaneously. Tissue ablation size and geometry is influenced by a variety of factors, such as the energy applicator design, number of energy applicators used simultaneously, ablation time and wattage, and tissue characteristics.

In operation, microwave energy having a wavelength, lambda (λ), is transmitted through the antenna assembly 12, e.g., along the proximal and distal radiating portions 140, 105, and radiated into the surrounding medium, e.g., tissue. The length of the antenna for efficient radiation may be dependent on the effective wavelength λeff that is dependent upon the dielectric properties of the medium being radiated. Antenna assembly 12 through which microwave energy is transmitted at a wavelength λ may have differing effective wavelengths λ_{eff} depending upon the surrounding medium, e.g., liver tissue as opposed to breast tissue.

Referring to FIG. 2, an embodiment of the antenna assembly 12 of FIG. 1 is shown and includes an inner conductor 210, an outer conductor 260, and may include a first dielectric material 240 separating the inner conductor 210 and the outer conductor 260. In some embodiments, the inner conductor 210 is formed from a first electrically conductive material (e.g., stainless steel) and the outer conductor 260 is formed from a second electrically conductive material (e.g., copper). In some embodiments, the outer conductor 260 coaxially surrounds the inner conductor 210 along a distal portion of the antenna assembly 12. Inner conductor 210 and the outer conductor 260 may be formed from any suitable electrically conductive material.

First dielectric material 240 may be formed from any suitable dielectric material, including, but not limited to, ceramics, water, mica, polyethylene, polyethylene terephthalate, polyimide, polytetrafluoroethylene (a.k.a. PTFE or Teflon^{®}, manufactured by E. I. du Pont de Nemours and Company of Wilmington, Delaware, United States), glass, or metal oxides. Antenna assembly 12 may be provided with a second dielectric material 290 surrounding the outer conductor 260 and/or the puck 130, or portions thereof. Second dielectric material 290 may be formed from any suitable dielectric material. In some embodiments, the second dielectric material 290 is formed from a material with a dielectric constant different than the dielectric constant of the first dielectric material 240.

In some embodiments, the antenna assembly 12 includes a conductor end portion 280, which may be formed from any suitable electrically conductive material. In some embodiments, the conductor end portion 280 is coupled to the inner conductor 210 and may be formed of the same material as the inner conductor 210. As shown in FIG. 2, the conductor end portion 280 may be spaced apart from the outer conductor 260 by the puck 130 disposed therebetween. Tapered region 120, or portions thereof, may surround a proximal portion of the conductor end portion 280. In some embodiments, the conductor end portion 280 is substantially cylindrically shaped, and may be formed from stainless steel. The shape and size of the conductor end portion 280 may be varied from the configuration depicted in FIG. 2. In some embodiments, at least a portion of the conductor end portion 280 is surrounded by the second dielectric material 290.

FIG. 4 illustrates an embodiment of a state diagram in accordance with the present disclosure that describes behavior of a handle user interface with respect to power on/off status, fluid circulation status, radiation detector status, and reflected power status. Presently disclosed handle user interface embodiments that may operate in accordance with the state diagram of FIG. 4 include the handle user interface 200 of FIG. 1. In the ablation device embodiment illustrated in FIG. 1, for example, the handle user interface 200 includes a power on/off switch 21, a fluid-flow monitoring system 26, a radiation detector 24, and a reflected-power monitoring system 23, and an intensity controller 25. Ablation devices configured with a handle user interface according to other embodiments of the present disclosure are shown in FIGS. 5, 6 and 8, as described later in this disclosure. Although power on/off status is described below in terms of microwave (MW) power, ablation device embodiments described by the state diagram of FIG. 4 may be implemented using electromagnetic radiation at microwave frequencies or at other frequencies.

As shown in FIG. 4, the action 421 of activating the power on/off switch transitions the presently disclosed ablation device to a power-off state "S1" if it is determined that the MW power is currently on when the power on/off switch is activated, or alternatively, results in a transition to a power-on state "S2" if it is determined that the MW power is currently off when the power on/off switch is activated. In the power-off state "S1 ", according to embodiments of the present disclosure, MW power is off, the green LED (e.g., 221 shown in FIG. 1) is off, and the red LED (e.g., 222 shown in FIG. 1) is illuminated. ln the power-on state "S2", MW power is on, the green LED is illuminated, and the red LED is off. As indicated by the dashed lines in FIG. 4, the action 421 of activating the power on/off switch may result in a transition out of the power-off state "S1" if it is determined that the switch activation turns MW power on, or alternatively, may result in a transition out of the power-on state "S2" if it is determined that the switch activation turns MW power off.

When the ablation device is in the power-on state "S2", a power-level-control sample 525 may be used to determine whether a power-level change has been input, e.g., by user operation of the intensity controller 25. ln the case where it is determined that the power level has been incremented, MW power may be increased, for example. It is contemplated that visual, auditory and/or haptic feedback may be associated with an increase in the power level and/or a decrease in the power level.

An unintentional radiation exposure may occur if an ablation device is not properly positioned to tissue and the probe is energized, or if the probe is wholly or partially removed from tissue while the probe is the energized state. According to embodiments of the present disclosure, a radiation-detector sample 424 may be used to determine whether radiation is detected. Handle user interface embodiments (e.g., 200, 500 and 600 shown in FIGS. 1, 5 and 6, respectively) are adapted to transition the ablation device to the power-off state "S1" in the case where it is determined that radiation is detected, e.g., to ensure patient and user safety.

According to embodiments of the present disclosure, a reflected power, P_{ref}, sample 423 may be used to determine whether power signals reflected from the probe 100 are detected. As shown in FIG. 4, based on the comparison result of a comparison of P_{ref} to a predetermined threshold value, the handle user interface may cause the ablation device to transition to the power-off state "S1". The predetermined threshold value may be obtained from a look-up table, e.g., based on a choice of electrosurgical instruments/attachments.

FIG. 5 shows an ablation device 500 according to an embodiment of the present disclosure that is similar to the ablation device 110 of FIG. 1, except for the shape of the handle body 530, the configuration of the power on/off switch 521, and the configuration of the indicator unit 520. Ablation device 500 includes the controller 20, the radiation detector 24, the reflected-power monitoring system 23, and the intensity controller 25, and the first and second LEDs 221, 222 of FIG. 1, and further description thereof is omitted in the interests of brevity.

Indicator unit 520 is generally adapted to provide a visual sensory alarm. In the embodiment illustrated in FIG. 5, the indicator unit 520 includes the first LED 221 and the second LED 222 in a row configuration disposed on a top, proximal portion of the handle body 533. The shape, size and location of the first and second LEDs 221, 222 may be varied from the configuration depicted in FIG. 5. Indicator unit 520 may additionally, or alternatively, be adapted to provide audio and/or other perceptible sensory alerts.

Power on/off switch 521 may be any suitable switch that generally fulfills the purpose of switching electrical circuits on and off or switching over from one electrical circuit to another. In the embodiment illustrated in FIG. 5, the power on/off switch 521 is a rocker-type switch that generally includes two wing portions projecting from opposite sides of a rotational axis for alternatingly engaging depressible operators of the switch 521.

FIG. 6 shows an ablation device 600 according to an embodiment of the present disclosure that is similar to the ablation device 110 of FIG. 1, except for the shape of the handle body 630, the configuration of the power on/off switch 621, and the configuration of the indicator unit 620. Ablation device 600 includes at least the radiation detector 24, the intensity controller 25, and the first and second LEDs 221, 222 of FIG. 1, and further description thereof is omitted in the interests of brevity.

As shown in FIG. 6, the indicator unit 620 may include the first LED 221 and the second LED 222 of FIG. 1 and a third LED 623. In some embodiments, a change in color of the third LED 623 may be used to indicate an abnormal fluid circulation condition. In the embodiment illustrated in FIG. 6, the power on/off switch 621 is a slider-type switch. The shape, size and location of the power on/off switch 621 may be varied from the configuration depicted in FIG. 6.

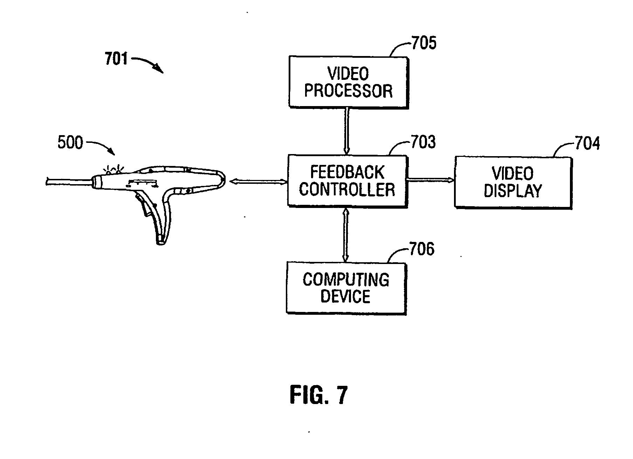

Embodiments of the present disclosure may include a feedback control system 701 as shown in FIG. 7. The feedback control system 701 includes a feedback controller 703. An ablation device (e.g., 500 shown in FIG. 5) provided with a data port (not shown) is connected to the feedback controller 703 via the data port, which may be wired (e.g., FireWire®, USB, Serial RS232, Serial RS485, USART, Ethernet, etc.) and/or wireless (e.g., Bluetooth^{®}, ANT3^{®}, KNX^{®}, Z-Wave^{®}, X10^{®}, Wireless USB, Wi-Fi^{®}, IrDA^{®}, NanoNet^{®}, TinyOS^{®}, ZigBee^{®}, 802.11 IEEE, and other radio, infrared, UHF, VHF communications and the like).

With reference to FIG. 7, the feedback controller 703 is configured to store the data transmitted by the ablation device to the feedback controller 703 as well as process and analyze the data. Feedback controller 703 is also connected to other devices, such as a video display 704, a video processor 705 and a computing device 706, e.g., a personal computer, a personal digital assistant (PDA), a smartphone, a storage device, etc. Video processor 705 is adapted to process output data generated by the feedback controller 603 for output on the video display 704. Computing device 706 is adapted for processing of the feedback data. ln one embodiment, the results of the sensor feedback analysis performed by the controller 524 may be stored internally for later retrieval by the computing device 706.

Examples of feedback controller embodiments are disclosed in commonly assigned U.S. Patent Application Serial No. 12/189,834 filed on August 12, 2008, entitled "POWERED SURGICAL STAPLING DEVICE".

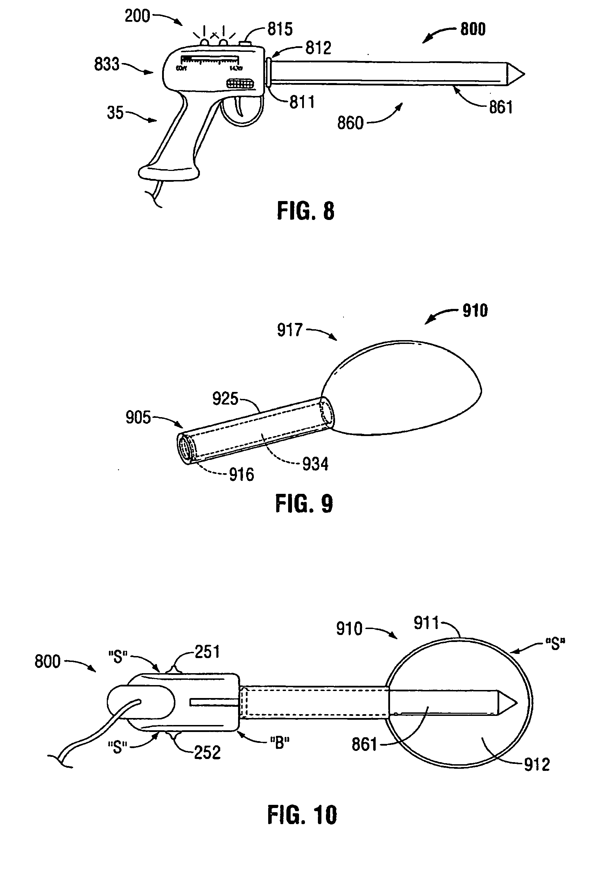

FIG. 8 shows an ablation device 800 according to an embodiment of the present disclosure that is similar to the ablation device 110 of FIG. 1, except for the shape of the handle body 833 and a male connector disposed at the proximal end of the probe 860. Ablation device 800 includes the handle user interface of FIG. 1, and further description thereof is omitted in the interests of brevity.

Handle body 833 is operably associated with the male connector 812. In embodiments, the male connector 812 includes a retainer member 811 that is movable between at least an engagement position and a released position. In embodiments, the handle body 833 may include a user operable switch 815, e.g., a push button, operable to move the male connector 812 from an engagement position, in which the retainer member 811 is engaged with a female connector (e.g., 916 shown in FIG. 9), to a released position, in which the retainer member 811 is disengaged from the female connector. The shape and size of the male connector 812 may be varied from the configuration depicted in FIG. 8.

FIG.9 shows an embodiment of a directional reflector assembly 910 in accordance with the present disclosure that includes a shell assembly 917, a tubular portion 930 defining a lumen 934, and a female connector 916 associated with the proximal end 905 of the tubular portion 930. Female connector 916 is adapted for engagement with the male connector 812 of the ablation device 800 of FIG. 8. FIG. 10 is a bottom view of the ablation device 800 of FIG. 8 shown with the directional reflector assembly 910 of FIG. 9 mounted on the probe 860 thereof.

Shell assembly 917, according to various embodiments, includes an outer portion 911 and an inner portion 912, and may include a recess defined in a planar surface "S" of the inner portion 912 generally configured to receive a distal portion 861 of probe 860 therein.

Outer portion 911 may include an electrically conductive material, such as, for example, copper, stainless steel, titanium, titanium alloys such as nickel-titanium and titanium-aluminum-vanadium alloys, aluminum, aluminum alloys, tungsten carbide alloys or combinations thereof. Portions of the outer portion 911 may be loaded with low- to mid-range permittivity dielectric materials to aid in radiation directivity and impedance matching. In general, the dielectric permittivity would increase in value with radial distance from the electrically-conductive member 911. Several shells, or other shapes, of different dielectric materials may nest together to form the outer portion 911.

Inner portion 912 may include a dielectric material. In some embodiments, the inner portion 912 includes dielectric material layers. For example, the inner portion 912 may include one or more thin layers, one or more thick layers or a mixture of thick and thin layers. Inner portion 912 may be composed of any suitable dielectric material which may be the same as, or different from, the dielectric material, if any, used in the outer portion 911. The dielectric materials used to form the inner portion 912 may vary in dielectric constant with shells or more complex dielectric layering to achieve the optimum antenna directivity and energy to tissue delivery. In embodiments, the dielectric material used to form the inner portion 912 may have a relatively high dielectric constant k (e.g., k = 80) to enhance the directional influence of the electromagnetic field.

Shell assembly 917 may be shaped in such a manner to provide a desired surface ablation shape as well as aid in impedance matching. Shell assembly 917 may have any suitable shape and may be designed for tight spaces encountered during surgical operations. Examples of shell assembly embodiments are disclosed in commonly assigned U.S. Patent Application Serial No. 12/568,524 filed on September 28, 2009, entitled "ELECTROSURGICAL DEVICES, DIRECTIONAL REFLECTOR ASSEMBLIES COUPLEABLE THERETO, AND ELECTROSURGICAL SYSTEMS INCLUDING SAME".

The above-described ablation devices with a user interface at the handle for treating tissue may be used to provide directional microwave ablation, wherein the heating zone may be focused to one side of the electrosurgical device, thereby allowing clinicians to target small and/or hard tumors without having to penetrate the tumor directly or affect more healthy tissue than necessary. The presently disclosed ablation devices with a user interface at the handle and directional reflector assemblies may allow clinicians to avoid ablating critical structures, such as large vessels, healthy organs or vital membrane barriers, by placing the electrosurgical device between the tumor and critical structure and directing the electromagnetic radiation toward the tumor and away from the sensitive structure.

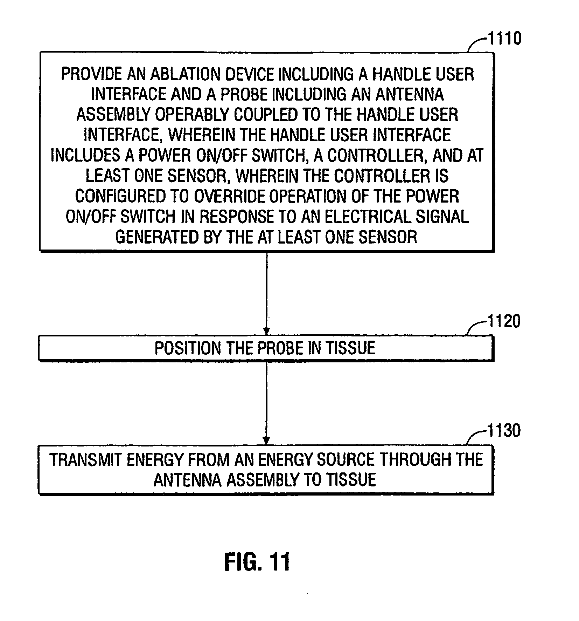

Hereinafter, a method of directing energy to tissue is described with reference to FIG. 11. It is to be understood that the steps of the method provided herein may be performed in combination and in a different order than presented herein without departing from the scope of the disclosure.

FIG. 11 is a flowchart illustrating a method of directing energy to tissue. In step 1110, an ablation device (e.g., 110 shown in FIGS. 1 and 3) is provided. The ablation device includes a handle user interface (e.g., 200 shown in FIG. 1) and a probe (e.g., 110 shown in FIG. 1) including an antenna assembly (e.g., 12 shown in FIGS. 1 and 2) operably coupled to the handle user interface. The handle user interface includes a power on/off switch (e.g., 21 shown in FIG. 1), a controller (e.g., 20 shown in FIG. 1), and at least one sensor (e.g., radiation detector 24 shown in FIG. 1), wherein the controller is configured to override operation of the power on/off switch in response to an electrical signal generated by the at least one sensor. The controller may be configured to override operation of the power on/off switch by transitioning the ablation device to a power-off state (e.g., "S1" shown in FIG. 4).

In step 1120, the ablation device (e.g., 110 shown in FIG. 1) is positioned to tissue. The ablation device may be inserted directly into tissue, inserted through a lumen, e.g., a vein, needle or catheter, placed into the body during surgery by a clinician, or positioned in the body by other suitable methods. The ablation device may be configured to operate with a directional radiation pattern.

In step 1130, energy is transmitted from an energy source (e.g., shown in FIG. 1) through the antenna assembly (e.g., 12 shown in FIGS. 1 and 2) to tissue. The energy source may be any suitable electrosurgical generator for generating an output signal. In some embodiments, the energy source is a microwave energy source, and may be configured to provide microwave energy at an operational frequency from about 500 MHz to about 10 GHz. In some embodiments, the energy source supplies power having a selected phase, amplitude and frequency.

## Claims

1. An ablation device (110), comprising:
a handle assembly (30) including a distal end (3);
an ablation probe (100) extending distally from the distal end (3) of the handle assembly (33);
a user interface (200) disposed at the handle assembly (30), wherein the ablation probe (100) is operably coupled to the user interface (200);
the user interface (200) including:
a controller unit (20);
a power on/off switch (21) operably coupled to the controller unit (20); and
at least one detector (24-26) capable of generating an electrical signal, wherein the controller unit (20) is in communication with the at least one detector (24) and configured to override operation of the power on/off switch (21) in response to the electrical signal generated by the at least one detector (24),
**characterized in that** the user interface (200) includes a fluid-flow monitoring system (26) configured to monitor fluid flow to the ablation probe (100), wherein
the at least one detector comprises a flow sensor of the fluid-flow monitoring system.

2. The ablation device of claim 1, wherein the controller unit (20) is configured to override operation of the power on/off switch (21) by transitioning the ablation device to a power-off state (S1).

3. The ablation device of claim 1, wherein the at least one detector comprises a radiation detector (24).

4. The ablation device of claim 1, wherein the at least one detector comprises a power detector configured to detect reflected power (P_{ref})

5. The ablation device of claim 1, wherein the user interface further includes an intensity controller (23) configured to adjust power parameters.

6. The ablation device of claim 1, wherein the user interface further includes an indicatory unit (22).

7. The ablation device of claim 1, wherein the ablation device is configured to operate with a directional radiation pattern.

8. The ablation device of claim 7, wherein ablation device includes a directional reflector assembly.

9. A system for ablating tissue, comprising:
an energy source (48) connected to the ablation device (110) according to claim 1.

10. The system of claim 9, further comprising a feedback control system (701) including a feedback controller (703) in communication with the ablation device.

11. The system of claim 9, wherein the ablation device (110) is configured to operate with a directional radiation pattern.

12. The system of claim 9, wherein the energy source (48) is an electrosurgical generator configured to supply power having a selected phase, amplitude and frequency.

## Patentansprüche

1. Ablationsvorrichtung (110) mit
einer Griffanordnung (30), die ein distales Ende (3) aufweist;
einer Ablationssonde (100), die sich vom distalen Ende (3) der Griffanordnung (33) distal erstreckt;
einer Benutzerschnittstelle (200), die an der Griffanordnung (30) angeordnet ist, wobei die Ablationssonde (100) betriebsmäßig mit der Benutzerschnittstelle (200) gekoppelt ist;
wobei die Benutzerschnittstelle (200) aufweist:
eine Steuereinheit (20);
einen Ein/Aus-Schalter (21), der betriebsmäßig mit der Steuereinheit (20) gekoppelt ist; und
wenigstens einen Detektor (24-26), der ein elektrisches Signal zu erzeugen vermag,
wobei die Steuereinheit (20) in Verbindung mit dem wenigstens einen Detektor (24) steht und dazu ausgebildet ist, die Funktion des Ein/Aus-Schalters (21) auf das von dem wenigstens einen Detektor (24) erzeugte elektrische Signal hin aufzuheben,
**dadurch gekennzeichnet, dass** die Benutzerschnittstelle (200) ein Flüssigkeitsströmung-Überwachungssystem (26) aufweist, das dazu ausgebildet, die Flüssigkeitsströmung zu der Ablationssonde (100) zu überwachen,
wobei der wenigstens eine Detektor einen Strömungssensor des Flüssigkeitsströmung-Überwachungssystems aufweist.

2. Ablationsvorrichtung nach Anspruch 1, wobei die Steuereinheit (20) dazu ausgebildet ist, die Funktion des Ein/Aus-Schalters (21) durch Überführen der Ablationsvorrichtung in einen ausgeschalteten Zustand (S1) aufzuheben.

3. Ablationsvorrichtung nach Anspruch 1, wobei der wenigstens eine Detektor einen Strahlungsdetektor (24) aufweist.

4. Ablationsvorrichtung nach Anspruch 1, wobei der wenigstens eine Detektor einen zum Erfassen reflektierter Energie (P_{ref}) ausgebildeten Energiedetektor aufweist.

5. Ablationsvorrichtung nach Anspruch 1, wobei die Benutzerschnittstelle ferner einen zum Einstellen von Energieparametern ausgebildeten Intensitätsregler (23) aufweist.

6. Ablationsvorrichtung nach Anspruch 1, wobei die Benutzerschnittstelle ferner eine Anzeigeeinheit (22) aufweist.

7. Ablationsvorrichtung nach Anspruch 1, wobei die Ablationsvorrichtung dazu ausgebildet ist, mit einer Richtstrahlcharakteristik betrieben zu werden.

8. Ablationsvorrichtung nach Anspruch 7, wobei die Ablationsvorrichtung eine Richtreflektoranordnung aufweist.

9. System zur Ablation von Gewebe, mit
einer Energiequelle (48), die mit der Ablationsvorrichtung (110) nach Anspruch 1 verbunden ist.

10. System nach Anspruch 9, das ferner ein Rückkopplungsregelsystem (701) mit einem mit der Ablationsvorrichtung in Verbindung stehenden Rückkopplungsregler (703) aufweist.

11. System nach Anspruch 9, wobei die Ablationsvorrichtung (110) dazu ausgebildet ist, mit einer Richtstrahlcharakteristik betrieben zu werden.

12. System nach Anspruch 9, wobei die Energiequelle (48) ein elektrochirurgischer Generator ist, der dazu ausgebildet ist, Strom einer ausgewählten Phase, Amplitude und Frequenz zu liefern.

## Revendications

1. Dispositif d'ablation (110), comprenant :
un ensemble de poignée (30) incluant une extrémité distale (3) ;
une sonde d'ablation (100) s'étendant distalement à partir de l'extrémité distale (3) de l'ensemble de poignée (33) ;
une interface utilisateur (200) disposée sur l'ensemble de poignée (30), dans lequel la sonde d'ablation (100) est opérationnellement couplée à l'interface utilisateur (200) ;
l'interface utilisateur (200) incluant :
une unité de contrôleur (20) ;
un commutateur de mise sous/hors tension (21) opérationnellement couplé à l'unité de contrôleur (20) ; et
au moins un détecteur (24-26) apte à générer un signal électrique,
dans lequel l'unité de contrôleur (20) est en communication avec l'au moins un détecteur (24) et configuré pour annuler une opération du commutateur de mise sous/hors tension (21) en réponse au signal électrique généré par l'au moins un détecteur (24),
**caractérisé en ce que** l'interface utilisateur (200) inclut un système de surveillance d'écoulement de fluide (26) configuré pour surveiller un écoulement de fluide jusqu'à la sonde d'ablation (100),
dans lequel l'au moins un détecteur comprend un capteur d'écoulement du système de surveillance d'écoulement de fluide.

2. Dispositif d'ablation selon la revendication 1, dans lequel l'unité de contrôleur (20) est configuré pour annuler une opération du commutateur de mise sous/hors tension (21) en faisant passer le dispositif d'ablation en un état hors tension (S1).

3. Dispositif d'ablation selon la revendication 1, dans lequel l'au moins un détecteur comprend un détecteur de rayonnement (24).

4. Dispositif d'ablation selon la revendication 1, dans lequel l'au moins un détecteur comprend un détecteur de puissance configuré pour détecter une puissance réfléchie (P_{ref}).

5. Dispositif d'ablation selon la revendication 1, dans lequel l'interface utilisateur inclut en outre un contrôleur d'intensité (23) configuré pour ajuster des paramètres de puissance.

6. Dispositif d'ablation selon la revendication 1, dans lequel l'interface utilisateur inclut en outre une unité d'indication (22).

7. Dispositif d'ablation selon la revendication 1, dans lequel le dispositif d'ablation est configuré pour fonctionner avec un motif de rayonnement directionnel.

8. Dispositif d'ablation selon la revendication 7, dans lequel le dispositif d'ablation inclut un ensemble de réflecteur directionnel.

9. Système d'ablation de tissu, comprenant :
une source d'énergie (48) connectée au dispositif d'ablation (110) selon la revendication 1.

10. Système selon la revendication 9, comprenant en outre un système de contrôle de rétroaction (701) incluant un contrôleur de rétroaction (703) en communication avec le dispositif d'ablation.

11. Système selon la revendication 9, dans lequel le dispositif d'ablation (110) est configuré pour fonctionner avec un motif de rayonnement directionnel.

12. Système selon la revendication 9, dans lequel la source d'énergie (48) est un générateur électrochirurgical configuré pour alimenter une énergie ayant une phase, une amplitude et une fréquence sélectionnées.
